# EUROPÄISCHE PATENTSCHRIFT

(11) **EP 4 070 094 B1**
(45) Veröffentlichungstag und Bekanntmachung des Hinweises auf die Patenterteilung: **02.07.2025**
(21) Anmeldenummer: 20816941.7
(22) Anmeldetag: 30.11.2020
(51) Int. Cl.: G01N 33/497

(54) **VERFAHREN ZUM BETREIBEN EINES ATEMGASANALYSEGERÄTS MIT WENIGSTENS EINEM GASSENSOR**
METHOD FOR OPERATING A RESPIRATORY GAS ANALYSIS DEVICE HAVING AT LEAST ONE GAS SENSOR
PROCÉDÉ DE FONCTIONNEMENT D'UN DISPOSITIF D'ANALYSE DE GAZ RESPIRATOIRE COMPORTANT AU MOINS UN CAPTEUR DE GAZ

(30) Priorität: 04.12.2019 DE 102019218849
(43) Veröffentlichungstag der Anmeldung: 12.10.2022
(73) Patentinhaber: Robert Bosch GmbH, 70442 Stuttgart (DE)
(72) Erfinder: BECK, Christoph, 73732 Esslingen (DE); JANK, Heike, 71394 Kernen Im Remstal (DE); HANAUER, Matthias Martin, 71229 Leonberg (DE); EIFLER, Georg, 71332 Waiblingen (DE); HAEBERLEN, Nino, 70569 Stuttgart (DE); MARTINEZ PRADA, Maria, 71134 Aidlingen (DE); SCHOEFER, Jan, 70376 Stuttgart (DE)
(86) Internationale Anmeldenummer: PCT/EP2020/083818
(87) Internationale Veröffentlichungsnummer: WO 2021/110564

(56) Entgegenhaltungen:
- WO-A1-2013/191633
- JP-A- 2019 184 571
- US-A1- 2020 393 443

## Beschreibung

Die vorliegende Erfindung betrifft ein Verfahren zum Betreiben eines Atemgasanalysegeräts mit wenigstens einem Gassensor, wobei das Atemgasanalysegerät wenigstens ein feuchteregulierendes Systemelement umfasst, das in einem schaltbaren, parallelen Luftführungspfad angeordnet ist. Weiterhin betrifft die Erfindung die Verwendung eines in einem schaltbaren, parallelen Luftführungspfad eines Atemgasanalysegeräts mit wenigstens einem Gassensor angeordneten feuchteregulierenden Systemelements zur Überprüfung des Feuchtezustands des Gassensors sowie ein entsprechend eingerichtetes Atemgasanalysegerät.

### Stand der Technik

Die von einem Menschen ausgeatmete Luft (Ausatemluft) enthält verschiedene medizinisch und insbesondere diagnostisch interessante Substanzen (Biomarker). So kann beispielsweise der Stickstoffmonoxidgehalt in der Ausatemluft untersucht werden, um Entzündungsprozesse oder chronische pulmonale Erkrankungen, wie z.B. Asthma bronchiale, erkennen zu können. Zur Analyse der Ausatemluft sind Atemgasanalysegeräte bekannt, die über entsprechende Sensoren, insbesondere Gassensoren, verfügen. Hierbei kommen vor allem NO- und/oder NO₂-Sensoren zum Einsatz, die für die Konzentrationsmessungen dieser Substanzen in der Ausatemluft genutzt werden. Derartige Gassensoren sind in der Regel empfindlich gegenüber Quereinflüssen, sodass sich beispielsweise variable Umgebungsfeuchtigkeiten und -temperaturen auf die Messgenauigkeit auswirken können. Somit kann es auch zu Lagereffekten an den Gassensoren kommen, wobei sich die Empfindlichkeit des Sensors mit andauernder Lagerung in Abhängigkeit von den Lagerbedingungen ändert und auf das Messsignal auswirkt. In diesem Zusammenhang spielt insbesondere die Feuchtigkeit, die sich am Sensor einstellt, eine Rolle.

JP2019184571 offenbart ein Atemgasanalysegerät mit Gassensoren und einem Feuchtesensor sowie einem feuchteregulierenden Element, wobei Gasproben über einen Luftführungspfad mit dem feuchteregulierenden Element und über einen Luftführungspfad ohne das feuchteregulierende Element geführt werden.

### Offenbarung der Erfindung

### Vorteile der Erfindung

Das vorgeschlagene Verfahren dient zum Betreiben eines Atemgasanalysegeräts mit wenigstens einem Gassensor, wobei der Feuchtezustand des Gassensors überprüft wird. Hierfür ist das Atemgasanalysegerät mit wenigstens einem feuchteregulierenden Systemelement ausgestattet, das in einem schaltbaren, parallelen Luftführungspfad angeordnet ist. Unter einem schaltbaren, parallelen Luftführungspfad ist hierbei zu verstehen, dass ein weiterer Luftführungspfad vorgesehen ist, der nicht über das feuchteregulierende Systemelement verläuft. Über diesen weiteren Luftführungspfad läuft in der Regel die Luftführung für die eigentliche gasanalytische Messung am Gassensor. Ein derartiges Atemgasanalysegerät kann beispielsweise für die Analyse von Stickstoffspezies eingerichtet sein und insbesondere mit NO-/NO₂-Sensoren ausgestattet sein. Bei dem Gassensor kann es sich beispielsweise um einen elektrochemischen Gassensor oder um einen Sensor auf der Basis von Feldeffekttransistoren (FET) handeln.

Zur Überprüfung des Feuchtezustands des Gassensors gemäß dem vorgeschlagenen Verfahren wird eine messbare Differenz bei einer Luftführung über den parallelen Luftführungspfad mit dem feuchteregulierenden Systemelement im Vergleich mit einer Luftführung über den Luftführungspfad ohne feuchteregulierendes Element ausgewertet. Mit diesem Verfahren wird das Problem gelöst, dass ein unbekannter Feuchtezustand am Gassensor die Messgenauigkeit des Gassensors derart beeinflussen kann, dass es zu ungenauen oder sogar falschen Messergebnissen kommt. Mit dem vorgeschlagenen Verfahren kann der Zustand des oder der Gassensoren und insbesondere deren Feuchtezustand bestimmt werden, sodass basierend auf diesen Ergebnissen gegebenenfalls Maßnahmen zur Wiederherstellung eines bestimmten Sensorzustands oder gegebenenfalls beispielsweise eine rechnerische Kompensation der derart erfassbaren Quereinflüsse eingeleitet werden können. Bei Anwendung des vorgeschlagenen Verfahrens werden aufwendige konstruktive Lösungen zur Abschirmung des oder der Gassensoren gegenüber Störeinflüssen aus der Umgebung, wie insbesondere der Feuchtigkeit, überflüssig. In diesem Zusammenhang sind bei bekannten Atemgasanalysegeräten beispielsweise Nafion-Schläuche bekannt, die die Feuchtigkeit der Gasprobe an die Umgebungsfeuchte anpassen. Auf derart aufwendige Lösungen, die im Allgemeinen auch nur schwer miniaturisierbar sind, kann bei Verwendung des vorgeschlagenen Verfahrens verzichtet werden, da mit dem vorgeschlagenen Verfahren das Atemgasanalysegerät gewissermaßen selbstständig den unter Umständen variablen Feuchtezustand des Gassensors erkennen kann, sodass daraus Maßnahmen abgeleitet werden können, um den Sensorzustand zu korrigieren oder Störgrößen gegebenenfalls zu kompensieren.

Das vorgeschlagene Verfahren generiert ein Signal, das auf einem Unterschied in der Feuchtigkeit bei einer Luftführung mit und ohne Feuchteregulation basiert. Der Feuchtezustand am Sensor wird dabei gewissermaßen auf indirektem Wege bestimmt, indem eine Gasprobe über das feuchteregulierende Systemelement (Feuchteregulator) geleitet wird, bevor sie auf den Gassensor trifft. Die durch das feuchteregulierende Systemelement veränderten Gaseigenschaften dienen der Generierung von Signalen bzw. Merkmalen, die Aufschluss über den Feuchtezustand im Bereich des Sensors geben. Somit wird nicht direkt der Feuchtezustand des Sensors gemessen, was im Allgemeinen sehr schwierig wäre, wenn gassensitive Schichten beispielsweise im Größenbereich von wenigen 100 nm Dicke verwendet werden. Es wird vielmehr mittels der Eigenschaften des Feuchteregulators auf den Feuchtezustand des Gassensors geschlossen, wobei eine Korrelation zwischen der Aufnahmekapazität des feuchteregulierenden Systemelements (beispielsweise von Aktivkohle) und dem Feuchtezustand des Sensors für die Zwecke der Erfindung genutzt wird. Wenn das Atemgasanalysegerät vor der Messung beispielsweise in sehr feuchter Umgebung gelagert wurde, gibt das feuchteregulierende Systemelement Feuchtigkeit an die Gasprobe ab. Wurde das Atemgasanalysegerät vor der Messung in trockener Umgebung gelagert, so entnimmt das feuchteregulierende Systemelement der Gasprobe Feuchtigkeit. Je nach Feuchtegehalt der Gasprobe und Feuchtezustand des feuchteregulierenden Systemelements wird die Gasprobe in ihrer Feuchtigkeit mehr oder weniger beeinflusst. Dieser Effekt ist direkt oder indirekt im Vergleich mit einer Luftführung ohne Feuchteregulation messbar Wenn also die Gasprobe zeitweise über das feuchteregulierende Systemelement und zeitweise nicht über das feuchteregulierende Systemelement geführt wird, ist die Differenz der unterschiedlichen Beeinflussung der Gasprobe durch das feuchteregulierende Systemelement nachweisbar. Je nachdem, welcher Luftführungspfad geschaltet ist, stellt sich eine andere Feuchte X am Gassensor ein. Im Moment des Umschaltens ergibt sich ein Feuchtesprung der Amplitude ΔX, mit Hilfe dessen auf den Feuchtezustand im Bereich des Gassensors rückgeschlossen werden kann.

Ein Umschalten zwischen dem Luftführungspfad mit feuchteregulierendem Systemelement und dem Luftführungspfad ohne feuchteregulierendem Systemelement kann beispielsweise durch gezielte Schaltung von entsprechenden Ventilen oder Pumpen durchgeführt werden. Entsprechende Anordnungen sind im Prinzip im Stand der Technik bekannt, wobei herkömmlicherweise derartige parallele Luftführungspfade genutzt werden, um durch entsprechende Systemelemente Störeinflüsse zu eliminieren und beispielsweise bestimmte Substanzen aus der Gasprobe zu entfernen und das Gas partiell aufzureinigen. Das vorgeschlagene Verfahren hingegen nutzt die parallelen Luftführungspfade, um eine indirekte Messung der Feuchtigkeit am Gassensor zu ermöglichen und so deren Einfluss auf die Messgenauigkeit kompensieren zu können oder um andere Maßnahmen einzuleiten.

In einer bevorzugten Ausgestaltung des vorgeschlagenen Verfahrens ist als feuchteregulierendes Systemelement ein Element vorgesehen, das Aktivkohle enthält. Neben der feuchteregulierenden Wirkung von Aktivkohle weist die Aktivkohle in der Regel auch eine gasfilternde Funktion auf. Diese gasfilternde Funktion von Aktivkohle wird in herkömmlichen Atemgasanalysegeräten bereits zur partiellen Gasreinigung genutzt. So wird in herkömmlichen Atemgasanalysegeräten die Aktivkohle beispielsweise genutzt, um eine erste Fraktion der Ausatemluft, die in der Regel störbehaftet ist, über die Aktivkohle zu leiten und dabei zu reinigen, sodass der Gassensor nicht mit Verunreinigungen belastet wird. In der Regel wird diese erste störbehaftete Fraktion des Ausatemgases für die eigentliche Atemgasanalyse verworfen, und erst nachfolgende Fraktionen werden im eigentlichen Sinne vermessen.

In bevorzugten Ausführungsformen kann die messbare Differenz bei der Luftführung mit und ohne Feuchteregulation auf der Basis von Messungen des Gassensors erfasst werden. Erfindungsgemäß ist das Atemgasanalysegerät mit einem separaten Feuchtesensor ausgestattet, der beispielsweise dem Gassensor vor- und/oder nachgeschaltet sein kann. Es können dabei gegebenenfalls auch mehrere Feuchtesensoren vorgesehen sein. Bei dem oder den Feuchtesensoren kann es sich beispielsweise um einen kapazitiven Feuchtesensor handeln. In Ausgestaltungen kann die messbare Differenz direkt über den oder die Feuchtesensoren gemessen werden. Weiterhin kann die messbare Differenz beispielsweise auch sowohl mit dem Gassensor als auch mit einem oder mehreren Feuchtesensoren erfasst werden.

In besonders bevorzugten Ausgestaltungen des vorgeschlagenen Verfahrens werden gegebenenfalls Maßnahmen eingeleitet, um den Feuchtezustand zu regulieren und/oder bei gasanalytischen Messungen zu kompensieren. Derartige Maßnahmen können beispielsweise automatisch eingeleitet werden oder der Anwender kann mit einem entsprechenden Signal oder einer Anzeige auf eine Notwendigkeit von derartigen Maßnahmen hingewiesen werden.

Bei den gegebenenfalls einzuleitenden Maßnahmen kann es sich beispielsweise um eine Sperrung des Atemgasanalysegeräts für weitere Messungen und/oder um eine Re-Kalibrierung des Atemgasanalysegeräts und/oder um eine Trocknung insbesondere des Gassensors und/oder um eine Trocknung des feuchteregulierenden Systemelements und/oder um eine Korrektur des Messwertes einer atemgasanalytischen Messung handeln. Die Einleitung von derartigen Maßnahmen kann beispielsweise, je nach der mathematischen Auswertemethode, von dem Über- oder Unterschreiten einer bestimmten Schwelle der messbaren Differenz abhängig gemacht werden. Das Über- oder Unterschreiten einer vorgebbaren Schwelle bei der messbaren Differenz weist dann darauf hin, dass eine bestimmte Feuchtigkeit im Bereich des Gassensors vorliegt, die das Messsignal beeinflusst, sodass nicht mehr von einer ausreichenden Messgenauigkeit oder gegebenenfalls sogar nicht mehr von der vollen Funktionsfähigkeit des Gassensors ausgegangen werden kann. Gegebenenfalls kann es bei der Auswertung auch vorgesehen sein, dass die messbare Differenz zwischen zwei vorgebbaren Schwellwerten liegen muss, um einen Feuchtezustand nachzuweisen, der eine ausreichende Messgenauigkeit gewährleistet.

Zweckmäßigerweise wird das vorgeschlagene Verfahren derart durchgeführt, dass die Überprüfung des Feuchtezustands regelmäßig erfolgt. Hierfür können beispielsweise bestimmte zeitliche Abstände vorgegeben werden, sodass die Betriebsbereitschaft mit einer ausreichenden Messgenauigkeit immer gewährleistet ist. Es kann beispielsweise auch vorgesehen sein, dass regelmäßig nach einer bestimmten Lagerzeit des Atemgasanalysegeräts eine Überprüfung durchgeführt wird.

Die Überprüfung des Feuchtezustands kann beispielsweise während bzw. zu Beginn einer regulären Atemgasmessung erfolgen, wobei die Ausatemluft des Probanden zeitweise über den parallelen Luftführungspfad mit dem feuchteregulierenden Systemelement und zeitweise über den Luftführungspfad ohne feuchteregulierendes Systemelement geführt wird, und die messbare Differenz entsprechend ausgewertet wird. Es kann auch vorgesehen sein, dass die Überprüfung des Feuchtezustands während einer sogenannten Pseudomessung erfolgt, indem insbesondere Umgebungsluft angesaugt und gemäß dem vorgeschlagenen Verfahren über die entsprechenden Luftführungspfade gelenkt wird. Allgemein wird unter einer Pseudomessung eine Messung mit dem Atemgasanalysegerät verstanden, bei der nicht Ausatemluft des Probanden, sondern Umgebungsluft in das Atemgasanalysegerät eingeleitet wird.

Schließlich umfasst die Erfindung ein Atemgasanalysegerät mit wenigstens einem Gassensor und wenigstens einem Feuchtesensor sowie wenigstens einem feuchteregulierenden Systemelement, das in einem schaltbaren, parallelen Luftführungspfad angeordnet ist. Dieses Atemgasanalysegerät ist dadurch gekennzeichnet, dass das Atemgasanalysegerät zur Durchführung des beschriebenen Verfahrens eingerichtet ist. Insbesondere ist dabei das Atemgasanalysegerät mit einer entsprechenden Steuerung ausgestattet, die eine Führung der Gasprobe über den Luftführungspfad mit feuchteregulierendem Systemelement und zeitlich davor oder danach eine Führung der Gasprobe über den Luftführungspfad ohne feuchteregulierendes Systemelement erlaubt. Prinzipiell ist es auch möglich, dass die Gasprobe zumindest zeitweise parallel über beide Luftführungspfade geleitet wird. Weiterhin ist eine entsprechende Auswertung der messbaren Differenz in der oben beschriebenen Weise vorgesehen, wobei diese Differenz auf Messungen des Gassensors oder auf Messungen des im Gerät vorgesehenen Feuchtesensors bei der Luftführung mit und ohne Feuchteregulation basiert.

Weitere Merkmale und Vorteile der Erfindung ergeben sich aus der nachfolgenden Beschreibung von Ausführungsbeispielen in Verbindung mit den Zeichnungen.

In den Zeichnungen zeigen:
- Fig. 1: Blockschaltbild des Gasflusses und beteiligter Systemkomponenten in einem Atemgasanalysegerät;
- Fig. 2: Darstellung des Feuchteverlaufs und des Feuchtesprungs ΔX beim Umschalten zwischen den Luftführungspfaden mit und ohne Feuchteregulation eines Atemgasanalysegeräts und
- Fig. 3: beispielhaftes Ablaufdiagramm der Durchführung des vorgeschlagenen Verfahrens.

### Beschreibung von Ausführungsbeispielen

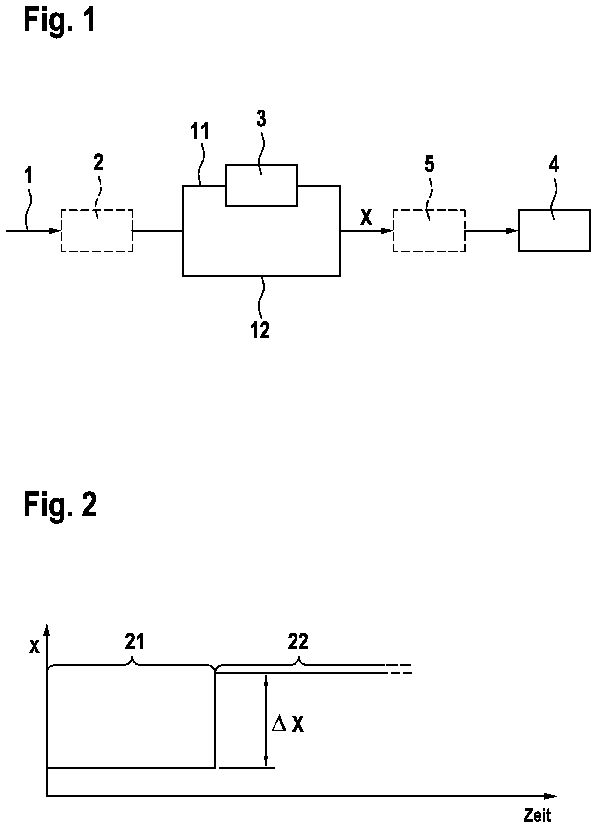

**Fig. 1** zeigt ein beispielhaftes Blockschaltbild für den Gasfluss in einem Atemgasanalysegerät mit den bei der Durchführung des vorgeschlagenen Verfahrens beteiligten Systemkomponenten. Der Pfeil 1 deutet hierbei die Ausatemluft des Probanden an, die über ein fakultatives Mundstück 2 in das Atemgasanalysegerät eingeleitet wird. Ein derartiges Mundstück 2 ist üblicherweise bei Atemgasanalysegeräten vorgesehen, um das Ausatmen in das Atemgasanalysegerät komfortabler zu gestalten. Weiterhin ist es oftmals vorgesehen, dass es sich bei dem Mundstück um einen Einweg-Artikel handelt, der aus hygienischen Gründen vor jeder Verwendung des Atemgasanalysegeräts ausgetauscht oder nur für einen bestimmten Probanden verwendet wird.

Innerhalb des Atemgasanalysegeräts verzweigt sich der Gasfluss in einen Luftführungspfad 11, der ein feuchteregulierendes Systemelement 3 (Feuchteregulator), das insbesondere Aktivkohle enthält, und in einen Luftführungspfad 12 ohne ein derartiges feuchteregulierendes Systemelement.

Bei dem Luftführungspfad 12 ohne feuchteregulierendes Element handelt es sich insbesondere um den eigentlichen Messpfad für die Gasprobe, über den die Gasprobe für die gasanalytische Untersuchung am Gassensor 4 geführt wird. Bei dem Gassensor 4 kann es sich beispielsweise um einen elektrochemischen Gassensor oder um einen Sensor auf der Basis von Feldeffekttransistoren (FET) handeln. Zwischen den Luftführungspfaden 11 und 12 kann über hier nicht näher dargestellte Mittel, beispielsweise über entsprechend schaltbare Ventile oder Pumpen, hin und her geschaltet werden. Nach einer anschließenden Wiedervereinigung der Luftführungspfade 11 und 12 gelangt die Gasprobe zu dem Gassensor 4, an dem die Messung der Analyten in der Gasprobe erfolgt. In diesem Ausführungsbeispiel ist dem Gassensor 4 der Feuchtesensor 5 vorgeschaltet. Bei dem Feuchtesensor 5 kann es sich beispielsweise um einen kapazitiven Feuchtesensor handeln. In anderen Ausgestaltungen kann der Feuchtesensor dem Gassensor 4 nachgeschaltet sein. In anderen Ausgestaltungen ist kein Feuchtesensor vorgesehen.

Für die Durchführung des vorgeschlagenen Verfahrens mit Überprüfung des Feuchtezustands wird in einer ersten Stufe zunächst in an sich bekannter Weise die Gasprobe dem Atemgasanalysegerät zugeführt. Hierbei kann die Gasprobe dem Gerät durch aktives Exhalieren des Probanden, das gegebenenfalls pumpenunterstützt ist, zugeführt werden. Bei einer sogenannten Pseudomessung, die ebenfalls für die Zwecke der Erfindung genutzt werden kann, wird die Gasprobe durch pumpengestütztes Ansaugen von Umgebungsluft zugeführt. Bei dem Eintritt der Gasprobe in das Gerät kann die Gasprobe zunächst mithilfe des optionalen Mundstücks 2, das beispielsweise mit Filtern und/oder Konvertern (beispielsweise zur Konversion von NO zu NO₂) ausgestattet ist, in ihren chemischen und/oder physikalischen Eigenschaften modifiziert werden. Je nach Aufbau, Zweck und Funktion des Atemgasanalysegeräts kann die Probe beispielsweise getrocknet oder befeuchtet und/oder oxidiert oder reduziert werden. Neben der Luftfeuchte können weitere

Eigenschaften der Gasprobe, wie zum Beispiel Temperatur, Druck oder ähnliches, beim Eintritt in das Gerät über geeignete Sensoren bestimmt werden. Hierbei kann es besonders vorteilhaft sein, anhand der gemessenen Luftfeuchte zu überprüfen, ob eine gewünschte Entfeuchtungsleistung eines verwendeten Mundstücks ausreichend ist. Ist die gemessene Entfeuchtungsleistung beispielsweise nicht ausreichend, kann der Nutzer zum Beispiel dazu aufgefordert werden, das Mundstück zu wechseln.

In einer zweiten Stufe erfolgt die Leitung der Gasprobe über das feuchteregulierende Systemelement 3 zur Durchführung der Überprüfung des Feuchtezustands am Gassensor. Hierfür wird die Gasprobe in dem Pfad 11 durch entsprechende Schaltung von Ventilen oder Pumpen über das feuchteregulierende Systemelement 3 geleitet. Hierdurch wird der Feuchtegehalt der Gasprobe beeinflusst. In bevorzugter Weise handelt es sich bei dem feuchteregulierenden Systemelement um einen Feuchteregulator mit Aktivkohle, welcher neben der Feuchteregulation auch eine gasfilternde Funktion aufweist, die auch für andere Zwecke eingesetzt werden kann. Wurde das Gerät vor der Messung in sehr feuchter Umgebung gelagert, so gibt die Aktivkohle Feuchtigkeit an die Gasprobe ab. Wurde das Gerät vor der Messung in trockener Umgebung gelagert, so entnimmt die Aktivkohle der Gasprobe Feuchtigkeit. Je nach Feuchtegehalt der Gasprobe und Feuchtezustand der Aktivkohle wird die Gasprobe in ihrem Feuchtegehalt mehr oder weniger beeinflusst. Die Feuchtigkeit der Gasprobe beim Eintreffen am Gassensor 4 wird in der Fig. 1 mit X angedeutet und lässt sich in der Regel als relative oder absolute Feuchte angeben. Für die Durchführung des vorgeschlagenen Verfahrens wird in dieser zweiten Stufe die Gasprobe zeitweise über den Pfad 11 und zeitweise über den Pfad 12, den eigentlichen Messpfad ohne Feuchteregulation, geführt. Hierbei ist es besonders zweckmäßig, die Gasprobe zunächst über den Pfad 11 und anschließend über den Pfad 12 zu führen, damit für die eigentliche gasanalytische Messung am Gassensor 4 nicht erneut umgeschaltet werden muss. In der Regel ist es hierbei ausreichend, wenn die Gasprobe jeweils für einige Sekunden zunächst über den Pfad 11 und dann über den Pfad 12 geleitet wird. Dies kann also auch während des Ausatemvorgangs des Probanden erfolgen (sofern nicht ohnehin für diesen Vorgang eine Pseudomessung vorgesehen ist). Prinzipiell ist es aber auch möglich, die Gasprobe zunächst über den Pfad 12 und anschließend über den Pfad 11 zu führen, um eine messbare Differenz zu erzeugen. Weiterhin kann innerhalb einer Messung mehrmals zwischen den Pfaden 11 und 12 hin- und hergeschaltet werden, um mehrere Feuchtesprünge auszuwerten. Je nachdem, welcher Pfad angesteuert wird, also der Pfad 11 über den Feuchteregulator 3 oder der Pfad 12 ohne Feuchteregulator, stellt sich eine andere Feuchte X im Bereich des Gassensors 4 ein. **Fig. 2** stellt diesen Verlauf der Feuchte X über die Zeit dar. In der ersten Phase 21 wird die Luft über den Pfad 11 geführt. Die Feuchte X ist in diesem Fall relativ niedrig. Nach dem Umschalten auf den weiteren Luftführungspfad 12 in der Phase 22 wird die Gasprobe an dem Feuchteregulator 3 vorbei geführt und es stellt sich dadurch ein Feuchtesprung der Amplitude ΔX ein. Auf der Basis von ΔX kann auf den Feuchtezustand des Gassensors rückgeschlossen werden. In dem hier dargestellten Beispiel steigt die Feuchte X bei Umschaltung auf den Pfad 12 sprunghaft auf einen höheren Wert an. Hieraus lässt sich schließen, dass das Gerät trocken gelagert wurde, da der Feuchteregulator der Gasprobe die Feuchte entziehen konnte. Wie die konkrete Auswertung des Feuchtesprungs erfolgt, ist abhängig von der Schaltrichtung der Luftführungspfade und der mathematischen Auswertemethode. Allgemein ist davon auszugehen, dass bei einer trockenen Lagerung des Geräts der Feuchteregulator seine volle Aufnahmekapazität besitzt, und dass daher ein deutlicher Feuchtesprung bei dem Umschalten zwischen den Luftführungspfaden zu erwarten ist. In diesem Fall ist der Feuchtezustand am Gassensor infolge der trockenen Lagerungsbedingungen in Ordnung.

In einer dritten Stufe erfolgt die eigentliche Bestimmung des Feuchtezustands am Gassensor. Hierbei wird zur Bewertung des Sensorzustands der Feuchtesprung ΔX vorzugsweise als Merkmal im Sensorsignal des Gassensors analysiert und in einen Feuchtezustand Z = f(ΔX) überführt. Dies kann beispielsweise mittels einer Transientenanalyse oder einer Fourier-Transformation des Signals erfolgen. Damit eine derartige Analyse des Sensorsignals des Gassensors erfolgen kann, sollte ein Gassensor mit einer Querempfindlichkeit gegenüber Feuchte mit einem kurzen Ansprechverhalten eingesetzt werden. In anderen Ausgestaltungen kann der Feuchtezustand des Sensors insbesondere auch mittels eines dedizierten Feuchtesensors 5 erfolgen, wobei der Feuchtesensor 5 dem Gassensor 4 vor- oder nachgeschaltet sein kann. Hierbei kann mit dem Feuchtesensor je nach Auswertungsmethode entweder direkt die Feuchte X oder der Feuchtesprung ΔX gemessen werden.

In einer vierten Stufe können gegebenenfalls erforderliche Korrektur- und/oder Kompensationsmaßnahmen eingeleitet werden, wenn der Feuchtezustand außerhalb vorgegebener Grenzen liegt. Wenn vorgebbare Grenzwerte für den Feuchtezustand insbesondere über- oder unterschritten werden, kann beispielsweise eine Sperrung des Geräts für weitere Messungen, eine Aufforderung zur Re-Kalibrierung des Geräts, eine Trocknung des Gassensors, beispielsweise durch aktives Ausheizen mittels eines gegebenenfalls vorgesehenen integrierten Heizelements, oder eine Korrektur des Messwerts einer atemgasanalytischen Messung in Abhängigkeit von dem Feuchtezustand des Gassensors erfolgen.

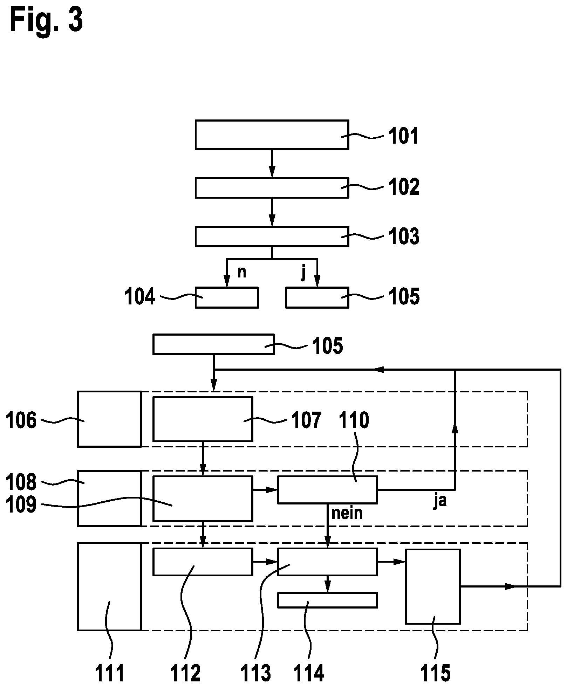

**Fig. 3** illustriert in beispielhafter Weise ein Ablaufdiagramm zur Erkennung und Korrektur des Feuchtezustands des Gassensors in einem Atemgasanalysegerät. Nach dem Einschalten 101 des Atemgasanalysegeräts erfolgt eine erste Überprüfung 102 des Geräts, wobei insbesondere abgefragt wird, ob eine maximal tolerierbare Lagerzeit überschritten ist, ob die Uhrzeit gesetzt ist und gegebenenfalls ein Batteriewechsel vorgenommen werden sollte, oder ob sonstige Parameter darauf hinweisen, dass das Gerät unter ungünstigen Umgebungsbedingungen gelagert wurde. Der Schritt 102 dient zur Überprüfung, ob das Gerät längere Zeit nicht genutzt wurde und sich daher in einem veränderten und/oder kritischen Feuchtezustand befinden könnte. Auf der Basis dieser Überprüfung 102 wird im Schritt 103 abgefragt, ob ein Feuchtigkeitscheck bzw. eine Überprüfung des Feuchtezustands des Gassensors erforderlich ist. Ist dies nicht der Fall, ist das Gerät messbereit (Schritt 104). Ergibt die Abfrage 103, dass ein Feuchtigkeitscheck erforderlich ist, wird die eigentliche Überprüfung des Feuchtezustands mit dem Schritt 105 gestartet. Der Start der Überprüfung des Feuchtezustands 105 ist im unteren Teil des Ablaufdiagramms der Fig. 3 nochmals separat dargestellt. Nach dem Start der Überprüfung 105 wird in einer ersten Phase in dieser Ausführungsform eine Pseudomessung 106 durchgeführt, indem Luft aus der Umgebung angesaugt wird. Hierbei wird der aktuelle Gerätezustand im Schritt 107 überprüft. In einer zweiten Phase 108 wird der Mundstückzustand im Schritt 109 überprüft und im Schritt 110 abgeleitet, ob ein neues Mundstück erforderlich ist. Ist dies der Fall, erfolgt nach einem Austausch des Mundstücks ein Rücksprung vor den Schritt 106. Ist dies nicht der Fall, wird in die dritte Phase 111 übergegangen. Gegebenenfalls kann direkt vom Schritt 109 in die dritte Phase 111 übergangen werden. In der dritte Phase 111 wird im Schritt 112 anhand von ΔX in der oben beschriebenen Weise analysiert wird, ob der Feuchtezustand des Gassensors vorgebbare Grenzwerte über- oder unterschreitet. Anhand dieser Auswertung wird im Schritt 113 abgefragt, ob daraus ableitbare Maßnahmen erforderlich sind, beispielsweise ein Ausheizen des Sensors. Ist dies nicht der Fall, kann im Schritt 114 der normale gasanalytische Messzyklus erfolgen. Ergibt die Abfrage im Schritt 113, dass Maßnahmen erforderlich sind, kann im Schritt 115 beispielsweise eine Reinigung des Gassensors zur Entfernung der störenden Feuchte oder ein Ausheizen des Sensors erfolgen. Alternativ zu derartigen feuchteregenerierenden Maßnahmen kann beispielsweise auch eine Korrektur des Messwerts zur Kompensation des Feuchteeinflusses bei einer nachfolgenden Messung oder eine Re-Kalibrierung oder gegebenenfalls eine Sperrung des Atemgasanalysegeräts für weitere Messungen erfolgen. Die Phasen 106, 108 und 111 können als Zyklus der Überprüfung des Sensorzustands wiederholt durchgeführt werden, beispielsweise auch, um die Effektivität einer Reinigung des Sensors im Schritt 115 zu überprüfen.

## Patentansprüche

1. Verfahren zum Betreiben eines Atemgasanalysegeräts nach längerer Lagerung, wobei das Atemgasanalysegerät wenigstens einen Gassensor (4) und wenigstens einen Feuchtesensor (5) sowie wenigstens ein feuchteregulierendes Systemelement (3) umfasst, das in einem Luftführungspfad (11) angeordnet ist, **dadurch gekennzeichnet, dass** der Feuchtezustand des Gassensors (4) überprüft wird, wobei eine Feuchtigkeit einer Gasprobe im Atemgasanalysegerät mit dem Feuchtesensor (5) bestimmt wird und indem eine gemessene Differenz der unterschiedlichen Beeinflussung der Feuchtigkeit derer Gasprobe bei einer Luftführung über den Luftführungspfad (11) mit dem feuchteregulierenden Systemelement (3) im Vergleich mit einer Luftführung über einen zum Luftführungspfad (11) parallelen Luftführungspfad (12) ohne feuchteregulierendes Element, über welchen die Luftführung bei einer gasanalytischen Messung erfolgt, ausgewertet wird.

2. Verfahren nach Anspruch 1, **dadurch gekennzeichnet, dass** das feuchteregulierende Systemelement (3) Aktivkohle enthält.

3. Verfahren nach Anspruch 1 oder Anspruch 2, **dadurch gekennzeichnet, dass** die Differenz auf der Basis von Messungen des Gassensors (4) erfasst wird.

4. Verfahren nach einem der vorhergehenden Ansprüche, **dadurch gekennzeichnet, dass** dem Gassensor (4) der Feuchtesensor (5) vor- und/oder nachgeschaltet ist.

5. Verfahren nach Anspruch 4, **dadurch gekennzeichnet, dass** die Differenz auf der Basis von Messungen des Feuchtesensors (5) erfasst wird.

6. Verfahren nach einem der vorhergehenden Ansprüche, **dadurch gekennzeichnet, dass** gegebenenfalls Maßnahmen eingeleitet werden, um den Feuchtezustand zu regulieren und/oder bei Messungen zur Atemgasanalyse zu kompensieren.

7. Verfahren nach Anspruch 6, **dadurch gekennzeichnet, dass** die gegebenenfalls einzuleitenden Maßnahmen wenigstens eine der folgenden Maßnahmen umfassen:
- Sperrung des Atemgasanalysegeräts für weitere Messung,
- Re-Kalibrierung des Atemgasanalysegeräts,
- Trocknung des Gassensors (4),
- Trocknung des feuchteregulierenden Systemelements (3) und
- Korrektur des Messwertes einer atemgasanalytischen Messung.

8. Verfahren nach einem der vorhergehenden Ansprüche, **dadurch gekennzeichnet, dass** die Überprüfung des Feuchtezustands regelmäßig erfolgt.

9. Verfahren nach einem der vorhergehenden Ansprüche, **dadurch gekennzeichnet, dass** die Überprüfung des Feuchtezustands während einer regulären Atemgasmessung erfolgt.

10. Verfahren nach einem der vorhergehenden Ansprüche, **dadurch gekennzeichnet, dass** die Überprüfung des Feuchtezustands während einer Pseudomessung erfolgt.

11. Atemgasanalysegerät mit wenigstens einem Gassensensor (4) und wenigstens einem Feuchtesensor (5) sowie wenigstens einem feuchteregulierenden Systemelement (3), das in einem vorzugsweise schaltbaren Luftführungspfad (11) angeordnet ist, **dadurch gekennzeichnet, dass** das Atemgasanalysegerät zur Durchführung eines Verfahrens gemäß einem der Ansprüche 1 bis 10 eingerichtet ist.

## Claims

1. Method for operating a respiratory gas analyser after prolonged storage, the respiratory gas analyser comprising at least one gas sensor (4) and at least one humidity sensor (5) and at least one humidity-regulating system element (3) that is arranged in an air conduction path (11), **characterized in that** the humidity state of the gas sensor (4) is checked, with a humidity of a gas sample in the respiratory gas analyser being determined using the humidity sensor (5) and by virtue of evaluating a measured difference in the different influences on the humidity of its gas sample in air conduction via the air conduction path (11) with the humidity-regulating system element (3) in comparison with air conduction via a humidity-regulating-element-free air conduction path (12) which is in parallel with the air conduction path (11) and via which the air flow is guided in a gas analytical measurement.

2. Method according to Claim 1, **characterized in that** the humidity-regulating system element (3) contains activated carbon.

3. Method according to Claim 1 or Claim 2, **characterized in that** the difference is acquired on the basis of measurements taken by the gas sensor (4).

4. Method according to any of the preceding claims, **characterized in that** the humidity sensor (5) is disposed upstream and/or downstream of the gas sensor (4).

5. Method according to Claim 4, **characterized in that** the difference is acquired on the basis of measurements taken by the humidity sensor (5).

6. Method according to any of the preceding claims, **characterized in that** measures for regulating the humidity state and/or for compensating for said humidity state in measurements for respiratory gas analysis are optionally initiated.

7. Method according to Claim 6, **characterized in that** the measures to be optionally initiated comprise at least one of the following measures:
- blocking the respiratory gas analyser from taking further measurements,
- recalibrating the respiratory gas analyser,
- drying the gas sensor (4),
- drying the humidity-regulating system element (3) and
- correcting the measured value of a respiratory gas analytical measurement.

8. Method according to any of the preceding claims, **characterized in that** the humidity state is checked regularly.

9. Method according to any of the preceding claims, **characterized in that** the humidity state is checked during a regular respiratory gas measurement.

10. Method according to any of the preceding claims, **characterized in that** the humidity state is checked during a pseudo-measurement.

11. Respiratory gas analyser having at least one gas sensor (4) and at least one humidity sensor (5) and at least one humidity-regulating system element (3) that is arranged in a preferably switchable air conduction path (11), **characterized in that** the respiratory gas analyser is configured to perform a method according to any of Claims 1 to 10.

## Revendications

1. Procédé pour faire fonctionner un appareil d'analyse de gaz respiratoire après un stockage prolongé, l'appareil d'analyse de gaz respiratoire comprenant au moins un capteur de gaz (4) et au moins un capteur d'humidité (5) ainsi qu'au moins un élément (3) de système de régulation de l'humidité qui sont agencés dans un chemin (11) de passage d'air, **caractérisé en ce que** l'état d'humidité du capteur de gaz (4) est contrôlé, l'humidité d'un échantillon de gaz dans l'appareil d'analyse de gaz respiratoire étant déterminée au moyen du capteur d'humidité (5) et en évaluant une différence mesurée entre les influences différentes sur l'humidité de cet échantillon de gaz lors d'un passage de l'air par le chemin (11) de passage d'air avec l'élément (3) de système de régulation de l'humidité et lors d'un passage de l'air par un chemin (12) de passage d'air parallèle au chemin (11) de passage d'air et dépourvu d'élément de régulation d'humidité, par lequel l'air est acheminé lorsqu'une mesure d'analyse de gaz a lieu.

2. Procédé selon la revendication 1, **caractérisé en ce que** l'élément (3) de système de régulation de l'humidité contient du charbon actif.

3. Procédé selon la revendication 1 ou la revendication 2, **caractérisé en ce que** la différence est détectée sur la base de mesures du capteur de gaz (4).

4. Procédé selon l'une des revendications précédentes, **caractérisé en ce que** le capteur d'humidité (5) est monté en amont et/ou en aval du capteur de gaz (4).

5. Procédé selon la revendication 4, **caractérisé en ce que** la différence est détectée sur la base des mesures du capteur d'humidité (5).

6. Procédé selon l'une des revendications précédentes, **caractérisé en ce que** des mesures sont prises, le cas échéant, pour réguler l'état d'humidité et/ou pour compenser lors des mesures destinées à l'analyse des gaz respiratoires.

7. Procédé selon la revendication 6, **caractérisé en ce que** les mesures à prendre le cas échéant comprennent au moins l'une des mesures suivantes :
- blocage de l'appareil d'analyse des gaz respiratoires pour d'autres mesures,
- ré-étalonnage de l'appareil d'analyse des gaz respiratoires,
- séchage du capteur de gaz (4),
- séchage de l'élément (3) de système de régulation de l'humidité, et
- correction de la valeur mesurée lors d'une mesure d'analyse des gaz respiratoire.

8. Procédé selon l'une des revendications précédentes, **caractérisé en ce que** la vérification de l'état d'humidité est effectuée régulièrement.

9. Procédé selon l'une des revendications précédentes, **caractérisé en ce que** le vérification de l'état d'humidité est effectué pendant une mesure régulière des gaz respiratoires.

10. Procédé selon l'une des revendications précédentes, **caractérisé en ce que** le contrôle de l'état d'humidité est effectué pendant une pseudo-mesure.

11. Appareil d'analyse de gaz respiratoire comprenant au moins un capteur de gaz (4) et au moins un capteur d'humidité (5) ainsi qu'au moins un élément (3) de système de régulation de l'humidité qui sont agencés dans un chemin (11) de passage d'air de préférence commutable, **caractérisé en ce que** l'appareil d'analyse de gaz respiratoire est conçu de manière à mettre en œuvre un procédé selon l'une des revendications 1 à 10.
